(19) Europäisches Patentamt
European Patent Office
Office européen des brevets

(11) EP 4 183 413 A1

(12) EUROPEAN PATENT APPLICATION
published in accordance with Art. 153(4) EPC

(43) Date of publication:
**24.05.2023 Bulletin 2023/21**

(21) Application number: **21841541.2**

(22) Date of filing: **19.07.2021**

(51) International Patent Classification (IPC):
***A61K 39/215*** (2006.01) ***A61P 31/14*** (2006.01)
***C07K 14/005*** (2006.01)

(52) Cooperative Patent Classification (CPC):
**A61K 39/215; A61P 31/14; C07K 14/005**

(86) International application number:
**PCT/KR2021/009290**

(87) International publication number:
**WO 2022/015124 (20.01.2022 Gazette 2022/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.07.2020 KR 20200088992**

(71) Applicants:
- **GeneOne Life Science, Inc.**
  **Seoul 06060 (KR)**
- **Korea Disease Control and Prevention Agency**
  **Chungcheongbuk-go 28159 (KR)**

(72) Inventors:
- **PARK, Young Keun**
  **Bluebell, Pennsylvania 19422 (US)**
- **CHO, Byung Mun**
  **Ganghwa-gun Incheon 23048 (KR)**
- **JEONG, Moon Sup**
  **Incheon 22009 (KR)**
- **LEE, Hyo Jin**
  **Sungnam-si Gyeonggi-do 13599 (KR)**
- **YEU, Song Yion**
  **Seoul 02831 (KR)**
- **CHO, Young Ran**
  **Seoul 03474 (KR)**
- **PARK, Bo Yeong**
  **Seoul 06103 (KR)**
- **GIL, Areum**
  **Anyang-si Gyeonggi-do 13944 (KR)**
- **JEON, Bo Hyun**
  **Incheon 21073 (KR)**
- **OH, Ye Eun**
  **Goyang-si Gyeonggi-do 10362 (KR)**
- **PARK, Gee Ho**
  **Seoul 04129 (KR)**
- **JOEL, Maslow**
  **Blue Bell, Pennsylvania 19422 (US)**
- **CHRISTINE, Claire Roberts**
  **Blue Bell, Pennsylvania 19422 (US)**
- **KIM, Mi Young**
  **Cheongju-si Chungcheongbuk-do 28159 (KR)**
- **LEE, Jung Ah**
  **Cheongju-si Chungcheongbuk-do 28159 (KR)**
- **LIM, Hee Ji**
  **Cheongju-si Chungcheongbuk-do 28159 (KR)**
- **HWANG, Yun Ho**
  **Cheongju-si Chungcheongbuk-do 28159 (KR)**

(74) Representative: **Potter Clarkson**
**Chapel Quarter**
**Mount Street**
**Nottingham NG1 6HQ (GB)**

<u>Remarks:</u>
The complete document including Reference Table(s) and the Sequence Listing(s) can be downloaded from the EPO website

# (54) VACCINE COMPOSITION FOR PREVENTING SEVERE ACUTE RESPIRATORY SYNDROME CORONAVIRUS 2 INFECTION

(57) The present invention relates to a vaccine composition for preventing severe acute respiratory syndrome coronavirus 2 infection. The vaccine composition for preventing severe acute respiratory syndrome coronavirus 2 infection according to the present invention comprises spike and ORF3a or nucleocapsid as an antigen against SARS-CoV-2. The vaccine composition has a significant effect of inducing antibody immune responses and T-cell immune responses by a plurality of co-expressed antigens compared to those comprising



Processed by Luminess, 75001 PARIS (FR)

(Cont. next page)

one antigen, and thus can be variously used in the field of prevention of severe acute respiratory syndrome coronavirus 2 infection.

【Figure 1a】

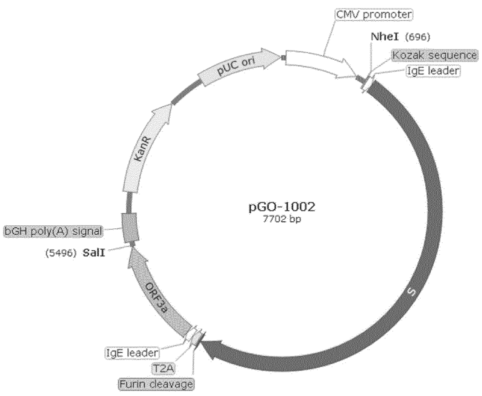

## Description

[Technical Field]

The present invention relates to a vaccine composition for preventing severe acute respiratory syndrome coronavirus 2 infection.

[Background Art]

Coronavirus disease-19 (COVID-19) is a respiratory infection caused by severe acute respiratory syndrome coronavirus 2 (SARS-CoV-2). SARS-CoV-2 is a new single-stranded RNA betacoronavirus newly discovered in Wuhan, China on December 23, 2019, and was named SARS-CoV-2 by the International Committee on Taxonomy of Viruses (ICTV) in January, 2020. The infection route of SARS-CoV-2 has not yet been clearly identified, but according to some studies, it has been reported that bats are infected with humans.
The genome of SARS-CoV-2 is a non-segmented form and encodes 10 proteins with a size of about 30 kb. The 10 proteins consist of 1 replicating polyproteins (open reading frames 1ab), 4 structural proteins (Spike, envelope, membrane and nucleocapsid) and 5 non-structural proteins (open reading frames 3a, 6, 7a, 8 and 10).
SARS-CoV-2 is contagious from person to person and has a quite high fatality rate, making it a major problem at home and abroad. On March 16, 2020, 167,511 patients occurred, of which 13,908 patients died, which was reported to WHO, and currently, the number of deaths is still rapidly increasing.
In Korea, the first SARS-CoV-2 infection was confirmed in a 36-year-old woman who entered Korea from China in January 2019, and until March 17, 2020, there were confirmed cases of SARS-CoV-2 infection, and 8,320 SARS-CoV-2 infections were confirmed, and 286,716 cases were isolated.
Since the first discovery in China in 2019, the outbreak has been concentrated in China, and in 2020, infected cases occurred in 160 or more countries including Korea, Japan, and Italy, and then SARS-CoV-2, which showed a high transmission rate, has problems accompanying complications such as pneumonia and acute renal failure as well as the respiratory system when infected and has a maj or problem due to its high mortality and contagiousness.
Considering that currently, new patients are continuously occurring at home and abroad and have a virus with a high transmission rate, it is urgent to develop a vaccine to prevent the virus infection.

[Disclosure]

[Technical Problem]

The present inventors made efforts to develop a vaccine for preventing severe acute respiratory syndrome coronavirus 2 infection and as a result, developed a DNA vaccine for expressing Spike and ORF3a or nucleocapsid and then completed the present invention.
Therefore, an object of the present invention is to provide a vaccine composition for preventing severe acute respiratory syndrome coronavirus 2 infection including (a) a SARS-CoV-2 Spike protein or a gene encoding the SARS-CoV-2 Spike protein, and (b) a SARS-CoV-2 ORF3a protein or a gene encoding the SARS-CoV-2 ORF3a protein; or a SARS-CoV-2 nucleocapsid or a gene encoding the SARS-CoV-2 nucleocapsid.
Another object of the present invention is to provide an immunogenic composition for severe acute respiratory syndrome coronavirus 2 including (a) a SARS-CoV-2 Spike protein or a gene encoding the SARS-CoV-2 Spike protein, and (b) a SARS-CoV-2 ORF3a protein or a gene encoding the SARS-CoV-2 ORF3a protein; or a SARS-CoV-2 nucleocapsid or a gene encoding the SARS-CoV-2 nucleocapsid.
Yet another object of the present invention is to provide a bicistronic expression cassette including (a) a gene encoding a SARS-CoV-2 Spike protein, and (b) a gene encoding a SARS-CoV-2 ORF3a protein; or a gene encoding a SARS-CoV-2 nucleocapsid protein.
Still another object of the present invention is to provide a vaccine composition for preventing severe acute respiratory syndrome coronavirus 2 infection including the bicistronic expression cassette.
Still yet another object of the present invention is to provide an immunogenic composition for preventing severe acute respiratory syndrome coronavirus 2 infection including the bicistronic expression cassette.
Still yet another object of the present invention is to provide a method for preventing severe acute respiratory syndrome coronavirus 2 infection including treating the bicistronic expression cassette to a subject.

[Technical Solution]

In order to achieve the object, the present invention provides a vaccine composition for preventing severe acute respiratory syndrome coronavirus 2 infection including (a) a SARS-CoV-2 Spike protein or a gene encoding the SARS-CoV-2 Spike protein, and (b) a SARS-CoV-2 ORF3a protein or a gene encoding the SARS-CoV-2 ORF3a protein; or a SARS-CoV-2 nucleocapsid or a gene encoding the SARS-CoV-2 nucleocapsid.

In addition, another aspect of the present invention provides an immunogenic composition for severe acute respiratory syndrome coronavirus 2 including (a) a SARS-CoV-2 Spike protein or a gene encoding the SARS-CoV-2 Spike protein, and (b) a SARS-CoV-2 ORF3a protein or a gene encoding the SARS-CoV-2 ORF3a protein; or a SARS-CoV-2 nucleocapsid or a gene encoding the SARS-CoV-2 nucleocapsid.

In addition, yet another aspect of the present invention provides a bicistronic expression cassette including (a) a gene encoding a SARS-CoV-2 Spike protein, and (b) a gene encoding a SARS-CoV-2 ORF3a protein; or a gene encoding a SARS-CoV-2 nucleocapsid protein.

In addition, still another aspect of the present invention provides a vaccine composition for preventing severe acute respiratory syndrome coronavirus 2 infection including the bicistronic expression cassette.

In addition, still yet another aspect of the present invention provides an immunogenic composition for preventing severe acute respiratory syndrome coronavirus 2 infection including the bicistronic expression cassette.

In addition, still yet another aspect of the present invention provides a method for preventing severe acute respiratory syndrome coronavirus 2 infection including treating the bicistronic expression cassette to a subject.

[Advantageous Effects]

The vaccine composition for preventing severe acute respiratory syndrome coronavirus 2 infection according to the present invention includes spike and ORF3a, or nucleocapsid as an antigen against SARS-CoV-2. The vaccine composition has a significant effect of inducing antibody immune responses and T-cell immune responses by a plurality of co-expressed antigens compared to those including one antigen, and thus can be variously used in the field of prevention of severe acute respiratory syndrome coronavirus 2 infection.

[Description of Drawings]

FIG. 1A is a diagram illustrating a cleavage map of a plasmid pGO-1002 including genes encoding antigens Spike and ORF3a according to the present invention.

FIG. 1B a diagram illustrating a post-translational modification mechanism of a peptide expressed by the plasmid pGO-1002.

FIG. 2A is a diagram illustrating results of confirming the production of antigen Spike and OPF3a proteins in cells transfected with the plasmid pGO-1002 through Western blotting.

FIG. 2B is a diagram illustrating results of confirming the production of antigen Spike and OPF3a proteins in cells transfected with the plasmid pGO-1002 through immunofluorescence staining.

FIG. 3 is a diagram illustrating results of evaluating the toxicity of the plasmid pGO-1002 according to the present invention in vivo.

FIG. 4 is a diagram illustrating results of measuring a titer of a Spike antigen-specific binding antibody through antibody immune responses.

FIG. 5 is a diagram illustrating results of measuring a titer of an ORF3a antigen-specific binding antibody through antibody immune responses.

FIG. 6 is a diagram illustrating results of evaluating T-cell immune responses of splenocytes stimulated with Spike OLP through ELISpot analysis.

FIG. 7 is a diagram illustrating results of evaluating T-cell immune responses of splenocytes stimulated with ORF3a OLP through ELISpot analysis.

FIG. 8 is a diagram illustrating results of evaluating T-cell immune responses of splenocytes stimulated with Spike OLP and ORF3a OLP through ELISpot analysis.

FIGS. 9 and 10 are diagrams illustrating results of evaluating a neutralizing antibody titer through a plaque reduction neutralization assay.

FIG. 11 is a diagram illustrating results of confirming a competitive binding ability with an ACE protein through ACE receptor competition evaluation.

[Best Mode of the Invention]

Hereinafter, the present invention will be described in detail.

According to an aspect of the present invention, the present invention provides a vaccine composition for preventing severe acute respiratory syndrome coronavirus 2 infection and an immunogenic composition for severe acute respiratory syndrome coronavirus 2, including (a) a SARS-CoV-2 Spike protein or a gene encoding the SARS-CoV-2 Spike protein, and (b) a SARS-CoV-2 ORF3a protein or a gene encoding the SARS-CoV-2 ORF3a protein; or a SARS-CoV-2 nucleocapsid or a gene encoding the SARS-CoV-2 nucleocapsid.

In an embodiment of the present invention, the gene encoding the Spike, ORF3a or nucleocapsid protein is preferably optimized with human codons.

In the present invention, Spike means a multifunctional protein that mediates the introduction of corona virus into host cells, and Spike of SARS-CoV-2 consists of 1273 amino acids (Genebank Accession No. YP_009724390.1). In addition, Spike has been used as an antigen of existing vaccines, but in large animals such as camels and monkeys, although clinical symptoms are improved, it has been confirmed that a virus removal effect (i.e., bactericidal effect) is at a low level. In the case of MERS-CoV, the same coronavirus, in the case of animal experiments using only Spike as an antigen, it has been confirmed that there is a limit to an effect of inducing immune responses in large animals compared to small animals such as mice. Accordingly, in order to induce an effective response in large animals, a need to supplement the response by adding antigens other than Spike has emerged.

In a preferred embodiment of the present invention, the gene encoding the Spike protein is optimized with human codons, and is preferably represented by a nucleotide sequence of SEQ ID NO: 1 or 3.

In the present invention, open reading frame 3a (ORF3a) is the largest accessory protein in SARS-CoV-2, consisting of 275 amino acids (Genebank Accession No. YP_009724391.1). The ORF3a is a protein involved in the internalization of SARS-CoV-2 during endocytosis.

In the present invention, an open reading frame (ORF) is an amino acid sequence and means a DNA nucleotide sequence to be translated.

In a preferred embodiment of the present invention, the gene encoding the ORF3a protein is optimized with human codons, and is preferably represented by a nucleotide sequence of SEQ ID NO: 2.

In the present invention, nucleocapsid refers to a protein structure containing RNA or DNA, which is genetic information of a virus, therein. The nucleocapsid consists of a 419-amino acid sequence (Genebank Accession No. YP_009724397.2).

In a preferred embodiment of the present invention, the gene encoding the nucleocapsid protein is optimized with human codons, and is preferably represented by a nucleotide sequence of SEQ ID NO: 4.

In an embodiment of the present invention, (a) the gene encoding the Spike protein; and (b) the gene encoding ORF3a or nucleocapsid protein are preferably included in a bicistronic expression vector, but is not limited thereto.

In the present invention, the vaccine is a drug that imparts acquired immunity to an antigen, and is administered for the purpose of inducing specific and active immunity against SARS-CoV-2.

In addition to Spike, ORF3a and nucleocapsid as antigens, the composition of the present invention may include one or more immune boosters or excipients or carriers suitable for constituting the composition.

The immune booster that may be included in the composition of the present invention refers to a substance that enhances the immune responses of an injected animal, and a plurality of different immune boosters is known to those skilled in the art. The immune boosters include Freund's complete and incomplete immune boosters, vitamin E, nonionic blocking polymers, muramyl dipeptide, Quil A, mineral oil and non-mineral oil, Carbopol, water-in-oil emulsion immune boosters, etc., but are limited thereto.

Examples of excipients that may be included in the composition of the present invention include aluminum phosphate, aluminum hydroxide, aluminum potassium sulfate and the like.

The carrier that may be included in the composition of the present invention may be a pharmaceutically acceptable carrier, and examples of the pharmaceutically acceptable carrier include saline, sterile water, a Ringer's solution, buffered saline, a dextrose solution, a maltodextrin solution, and glycerol, ethanol, etc.

The composition of the present invention may further include preservatives and other additives, for example, antimicrobial agents, antioxidants, chelating agents, inert gases, and the like. The preservatives include formalin, thimerosal, neomycin, polymyxin B, amphotericin B, and the like. The composition of the present invention may include one or more suitable emulsifiers, such as Span or Tween. In addition, the composition of the present invention may include a protecting agent, and protecting agents known in the art may be used without limitation, and may include Lactose (LPGG) or Trehalose (TPGG), but are not limited thereto.

In an embodiment of the present invention, the composition is preferably for twice doses, more preferably for twice doses at 2-week intervals.

In an embodiment of the present invention, it is preferred that the composition simultaneously induces the immunogenicity against (a) the Spike protein; and (b) the ORF3a or nucleocapsid protein.

In an embodiment of the present invention, it is preferred that the composition simultaneously induces antibody responses and T-cell immune responses.

According to yet another aspect of the present invention, the present invention provides a bicistronic expression cassette including (a) a gene encoding a SARS-CoV-2 Spike protein, and (b) a gene encoding a SARS-CoV-2 ORF3a protein; or a gene encoding a SARS-CoV-2 nucleocapsid protein.

According to still another aspect of the present invention, the present invention provides a vaccine composition for preventing severe acute respiratory syndrome coronavirus 2 infection and an immunogenic composition for preventing severe acute respiratory syndrome coronavirus 2 infection including the bicistronic expression cassette.

In an embodiment of the present invention, the gene encoding the Spike protein is preferably optimized with human codons, more preferably a signal peptide unique to SARS-CoV-2 replaced with an IgE leader sequence.

In a preferred embodiment of the present invention, the gene encoding the Spike protein may be represented by a nucleotide sequence of SEQ ID NO: 1 or 3.

In an embodiment of the present invention, the gene encoding the ORF3a protein is preferably optimized with human codons, more preferably added with an IgE leader sequence.

In a preferred embodiment of the present invention, the gene encoding the ORF3a protein may be represented by a nucleotide sequence of SEQ ID NO: 2.

In an embodiment of the present invention, the gene encoding the nucleocapsid protein is preferably optimized with human codons, more preferably added with an IgE leader sequence.

In a preferred embodiment of the present invention, the gene encoding the nucleocapsid protein may be represented by a nucleotide sequence of SEQ ID NO: 4.

In the present invention, bicistronic refers to a system in which a ribosome may synthesize a polypeptide even inside mRNA in a eukaryotic cell, so that multiple proteins may be synthesized from one mRNA, and in the present invention, expression cassettes were designed so that (i) Spike protein and ORF3a protein; or (ii) Spike protein and nucleocapsid protein may be expressed simultaneously.

In a preferred embodiment of the present invention, the expression cassette designed so that (i) the Spike protein and the ORF3a protein may be simultaneously expressed may include a gene encoding the Spike protein represented by the nucleotide sequence of SEQ ID NO: 1; and a gene encoding the ORF3a protein represented by the nucleotide sequence of SEQ ID NO: 2.

In a preferred embodiment of the present invention, the expression cassette designed so that (ii) the Spike protein and the nucleocapsid protein may be simultaneously expressed may include a gene encoding the Spike protein represented by the nucleotide sequence of SEQ ID NO: 3; and a gene encoding the nucleocapsid protein represented by SEQ ID NO: 4.

In the present invention, the expression cassette refers to a unit cassette that includes a promoter and a gene encoding a target protein and may be expressed to produce a target protein operably linked to the downstream of the promoter. Various factors capable of helping the efficient production of the target protein may be included inside or outside such an expression cassette. Specifically, in the target protein expression cassette, the gene encoding the target protein may be operably linked to the downstream of a promoter sequence. The 'operably linked' means that the gene sequence and the promoter sequence are functionally linked to each other so that a nucleic acid sequence having promoter activity of the present invention initiates and mediates the transcription of the gene encoding the target protein. The operable linkage may be prepared using genetic recombination techniques known in the art, and site-specific DNA cleavage and linkage may be prepared using cleavage and linkage enzymes in the art, but are not limited thereto.

In an embodiment of the present invention, it is preferred that the bicistronic expression cassette further includes one or more components selected from the group consisting of additional components, that is, a CMV promoter, a Kozak sequence, an IgE leader sequence, a furin recognition site, a GSG-T2A cleavage site, a BGH polyadenylation signal, a kanamycin resistance gene and a pUC origin. The CMV promoter, the Kozak sequence and the IgE leader sequence are for inducing high-level gene expression and secretion in animal cells; the furin recognition site and the GSG-T2A cleavage site are for protein cleavage; the BGH polyadenylation signal is for efficient transcription termination and transcript stabilization; the kanamycin resistance gene is an E. coli selectable marker; and the pUC Origin is for maintaining a high copy number in E. coli.

In an embodiment of the present invention, the bicistronic expression cassette is preferably a vector, more preferably represented by the following cleavage map.

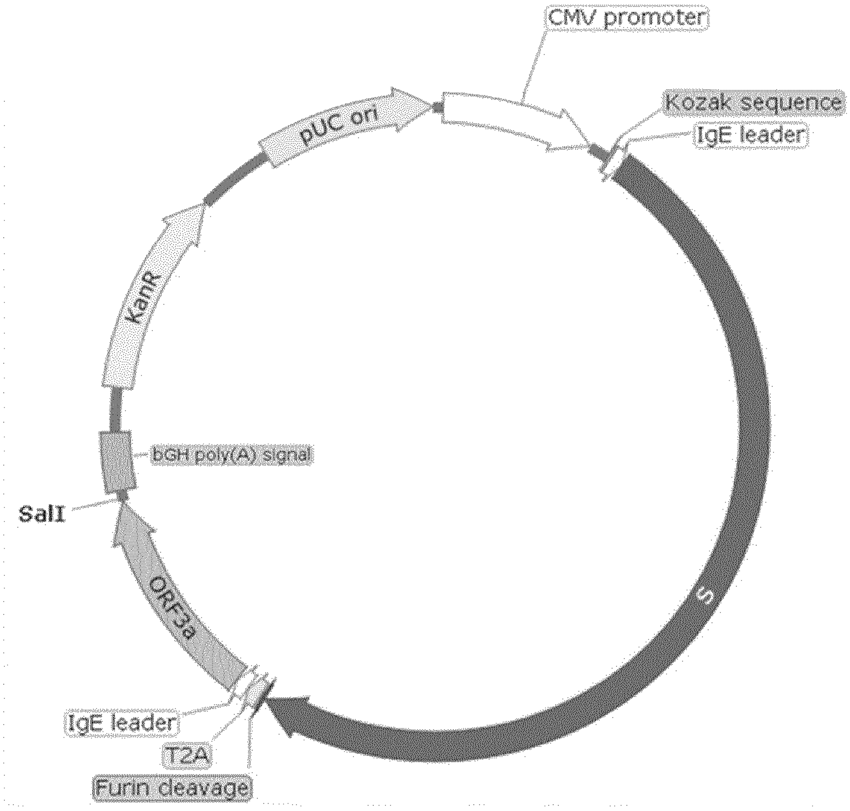


In the present invention, a vector means a means for expressing a target gene in a host cell. For example, the vector includes viral vectors such as a plasmid vector, an adenoviral vector, a retroviral vector, and an adeno-associated viral vector. The vector which may be used as the recombinant vector may be prepared by manipulating plasmids (e.g., pGLS, pSC101, pGV1106, pACYC177, ColE1, pKT230, ME290, pBR322, pUC8/9, pUC6, pBD9, pHC79, pIJ61, pLAFR1, pHV14, pGEX series, pET series, pUC19, etc.), phages (e.g., λgt4λB, λCharon, λΔz1, M13, etc.) or viruses (e.g., AAV, MVA, etc.), which have been often used in the art.

The recombinant vector may typically be constructed as a vector for cloning or a vector for expression. As the expression vector, conventional vectors used in the art to express foreign proteins in plants, animals, or microorganisms may be used. The recombinant vector of the present invention may be constructed through various methods known in the art.

The recombinant vector may be constructed using prokaryotic or eukaryotic cells as a host. For example, in the case of using the eukaryotic cell as the host, a replication origin operating in eukaryotic cell included in the vector includes an f1 replication origin, an SV40 replication origin, a pMB1 replication origin, an adeno replication origin, an AAV replication origin, a CMV replication origin, and a BBV replication origin, but is not limited thereto. In addition, a promoter (e.g., metallothionein promoter) derived from a genome of a mammalian cell or a promoter (e.g., an adenovirus late promoter, a vaccinia virus 7.5K promoter, a SV40 promoter, a cytomegalovirus (CMV) promoter and a tk promoter of HSV) derived from a mammalian virus may be used, and a polyadenylation sequence is generally included as a transcription termination sequence.

In an embodiment of the present invention, the composition is preferably administered 1 to 3 times at intervals of 1 to 3 weeks, more preferably inoculated 2 times at intervals of 2 weeks.

In an embodiment of the present invention, the composition may be formulated using methods known in the art to enable rapid release, or sustained or delayed release of active ingredients upon administration to a mammal. The formulation includes include powders, granules, tablets, emulsions, syrups, aerosols, soft or hard gelatin capsules, sterile injectable solutions, and sterile powder forms.

In an embodiment of the present invention, the composition may be administered by at least one selected from the group consisting of intramuscular, intravenous, subcutaneous, intradermal and intranasal routes.

In an embodiment of the present invention, the composition may be administered through a method of applying a physical stimulus to increase delivery efficiency during administration, and examples of the method include an electroporation method, a gene gun method, a jet injection method, and the like.

In the present invention, the electroporation is a technique for introducing chemicals, drugs or DNA into cells by increasing the permeability of a plasma membrane by applying an electric field to cells. In particular, the electroporation is used to transform bacteria, animal cells or plant cells by introducing new DNA.

In the present invention, the gene gun refers to a device made by attaching a recombinant plasmid to gold, tungsten or plastic particles of an appropriate size with a diameter of 1 to 5 μm and passing through a cell wall or cell membrane by accelerating at an appropriate speed. The delivery method using the gene gun is used to introduce a biological substance into living cells.

In the present invention, the jet injection method refers to a method of finely injecting a liquid drug under high pressure using an injection syringe without using an injection needle and injecting the liquid drug under the skin.

In a preferred embodiment of the present invention, the composition is preferably administered intramuscularly by electroporation.

In an embodiment of the present invention, the composition may be administered through a biochemical method to increase delivery efficiency, and preferably, the composition may be administered through a liposome-mediated delivery method.

In the present invention, the liposome-mediated delivery method refers to a method of delivering a target substance into cells using liposomes containing the target substance.

The composition according to the present invention may be formulated in a suitable form together with a generally used pharmaceutically acceptable carrier. The pharmaceutically acceptable carrier includes parenteral administration carriers such as water, suitable oil, saline, aqueous glucose and glycol, and the like, and may further include a stabilizer and a preservative. A suitable stabilizer includes antioxidants such as sodium hydrogen sulfite, sodium sulfite or ascorbic acid. A suitable preservative includes benzalkonium chloride, methyl- or propyl-paraben and chlorobutanol. In addition, the composition according to the present invention, if necessary according to the administration method or formulation, may appropriately include a suspending agent, a solubilizing agent, a stabilizer, an isotonic agent, a preservative, an anti-adsorption agent, a surfactant, a diluent, an excipient, a pH adjuster, a pain reliever, a buffer, an antioxidant and the like.

The dosage of the composition to a patient depends on many factors, including height, body surface area, age, particular compound to be administered, sex, time and route of administration, general health, and other drugs to be administered concurrently of a patient.

In addition, the composition of the present invention is administered in a therapeutically effective amount.

In the present invention, the therapeutically effective amount refers to an amount enough to treat diseases at a reasonable benefit/risk ratio applicable to medical treatment, and an effective dose level may be determined according to factors including a kind of subject, the severity, age, gender, the activity of a drug, sensitivity to a drug, a time of administration, a route of administration, an excretion rate, duration of treatment, and drugs to be simultaneously used, and other factors well-known in the medical field. The composition of the present invention may be administered at 0.01 mg/60 kg to 1 g/60 kg based on an adult of 60 kg. Meanwhile, the dosage may be appropriately adjusted according to the age, sex and condition of the patient.

According to the present invention, it was confirmed that the composition including a bicistronic expression cassette including genes encoding antigen proteins Spike and ORF3a; or a bicistronic expression cassette including genes encoding Spike and nucleocapsid effectively induces antigen-antibody immune responses and T-cell immune responses and has high neutralizing antibody titer and a competitive binding ability with a hACE2 protein. Therefore, the composition according to the present invention may be variously used in the field of prevention of SARS-CoV-2 virus infection.

According to still yet another aspect of the present invention, the present invention provides a method for preventing severe acute respiratory syndrome coronavirus 2 infection including treating the bicistronic expression cassette to a subject.

In an embodiment of the present invention, the bicistronic expression cassette is preferably treated in a subject in the form of a vector including the bicistronic expression cassette, but is not limited thereto.

In the present invention, the subject may be a subject having a risk of being infected with severe acute respiratory syndrome coronavirus 2 or a subject expected to be infected.

[Modes for the Invention]

Hereinafter, the present invention will be described in more detail through Examples. These Examples are just illustrative of the present invention, and it will be apparent to those skilled in the art that it is not interpreted that the scope of the present invention is limited to these Examples.

## Example 1. Selection of candidate antigen and design of bicistronic vector

Candidate antigens Spike (Genebank Accession No. YP_009724390.1), ORF3a (Genebank Accession No. YP_009724391.1) and nucleocapsid (Genebank Accession No. YP_009724397.2) were selected from the total sequence of Wuhan-Hu-1 (Genebank Accession No. MN908947).

Among the candidate antigens, ORF3a is the largest accessory protein consisting of 275 amino acids and is involved in internalization of SARS-CoV-2 during endocytosis.

Among the selected candidate antigens, a bicistronic vector (i.e., a combined vaccine in the form of a DNA vaccine) including a gene encoding (i) Spike and ORF3a; or (ii) Spike and nucleocapsid was prepared. The gene encoding Spike, ORF3a or nucleocapsid used in the preparation of the bicistronic vector is a nucleotide sequence obtained by optimizing an amino acid sequence of the corresponding Accession No. with human codons. In the case of Spike, a signal peptide unique to SARS-CoV-2 was replaced with an IgE leader sequence, and in the case of ORF3a and nucleocapsid, an IgE leader sequence was added. The gene encoding the Spike protein used in the production of the bicistronic vector is represented by a nucleotide sequence of SEQ ID NO: 1 or 3, the gene encoding the ORF3a protein is represented by a nucleotide sequence of SEQ ID NO: 2, and the gene encoding the nucleocapsid protein is represented by a nucleotide sequence of SEQ ID NO: 4.

A bicistronic vector (pGLS-BC-nCoV-S-Orf3a_hCO) including (i) a gene encoding a Spike protein (SEQ ID NO: 1) and a gene encoding ORF3a (SEQ ID NO: 2) was designed, which was named a plasmid pGO-1002. A cleavage map of the plasmid pGO-1002 was illustrated in FIG. 1A. A peptide expressed in cells using the plasmid pGO-1002 of FIG. 1A includes various cleavage sites such as signal peptidase and carboxypeptidase. As illustrated in FIG. 1B, the peptide expressed by the plasmid pGO-1002 is cleaved by post-translational modification, and thus only Spike and ORF3a proteins are secreted during extracellular secretion.

A bicistronic vector (pGLS-BC-nCoV-S-N_hCO) including (ii) a gene encoding a Spike protein (SEQ ID NO: 3) and a gene encoding nucleocapsid (SEQ ID NO: 4) was designed, which was named a plasmid pGO-1003. A peptide expressed in cells using the plasmid pGO-1003 includes various cleavage sites such as signal peptidase and carboxypeptidase. The peptide expressed by the plasmid pGO-1003 is cleaved by post-translational modification, and thus only Spike and nucleocapsid proteins are secreted during extracellular secretion.

In addition, in an experiment to be described below, a plasmid pGLS101 was used as a negative control, and plasmids pGLS-Spike (pGO-1001) and pGLS-ORF3a into which spike and ORF3a were inserted, respectively, were used as positive controls. The plasmid pGLS101 was derived from a pVAX1 plasmid (Cat. No. V26020) from Invitrogen Co., Ltd.

**Example 2. Confirmation of antigen production in vitro**

It was confirmed whether the plasmid pGO-1002 prepared in Example 1 produced antigen Spike and ORF3a proteins in transfected cells. Specifically, an HEK293T cell line was transfected with the plasmid pGO-1002 (pGLS-BC-nCoV-S-ORF3a_hCO). Cells were lysed after 48 hours of transfection. Production of the Spike and ORF3a proteins was confirmed by SDS-PAGE and Western blotting. Antibodies used in the Western blotting are as follows.

<Primary antibody>

- Anti-SARS-CoV-2 Spike antibody (Cat.3525, ProSci), 1 : 5,000
- Anti-ORF3a serum (Lab-made polyclonal Ab), 1 : 100
- Anti-β-actin (ab8226, Abeam), 1 : 1,000

<Secondary antibody>

- Anti-Rabbit IgG-HRP (Cat. G-21234, Thermo), 1 : 10,000
- Anti-Mouse IgG-HRP (62-6520, Thermo), 1 : 5,000

Western blotting results were illustrated in FIG. 2A.

As illustrated in FIG. 2A, it was confirmed that Spike and ORF3a were normally expressed in the cells transfected with the plasmid pGO-1002, respectively.

Expression of Spike and ORF3a proteins was confirmed by immunofluorescence staining. Specifically, the plasmid pGO-1002 was transfected into the HEK293T cell line. The transfected cells were immunofluorescently labeled after 48 hours of transfection. DNA in cell nuclei was stained and labeled with DAPI and used as a control (blue panel). The staining results were shown in FIG. 2B.

As illustrated in FIG. 2B, it was confirmed that Spike (TRITC) and ORF3a (FITC) were normally expressed in the cells transfected with the plasmid pGO-1002, respectively.

In addition, the experiment was performed in the same manner as above using the bicistronic vector pGO-1003 including the genes encoding Spike and nucleocapsid prepared in Example 1.

As a result, it was confirmed that Spike and nucleocapsid were normally expressed in the cells transfected with the bicistronic vector including the genes encoding Spike and nucleocapsid prepared in Example 1, respectively.

**Example 3. Evaluation of antigen toxicity**

The toxicity of the plasmid pGO-1002 prepared in Example 1 was evaluated. In the cell evaluation, the toxicity was evaluated by measuring a body weight change induced by antigen expression in mice immunized with the plasmid pGO-1002. The immunized mice were intramuscularly inoculated with the plasmid pGO-1002 through electroporation inoculation, and inoculated twice at 2-week intervals. The mice used in Example were first inoculated with the plasmid pGO-1002, and then second inoculated 15 days later. A negative control was inoculated with a plasmid pGLS-101, and a positive control was inoculated with a plasmid pGLS-ORF3a. The results of evaluating the toxicity of the antigen were illustrated in FIG. 3.

As illustrated in FIG. 3, an experimental group (pGLS-Spike-ORF3a) inoculated with the plasmid pGO-1002 showed a similar weight increased rate to the negative and positive controls. These results indicate that the antigens Spike and ORF3a have low toxicity in vivo.

In addition, the experiment was performed in the same manner as above using the bicistronic vector pGO-1003 including the genes encoding Spike and nucleocapsid prepared in Example 1.

As a result, the experimental group showed a similar weight increased rate to the negative and positive controls, which means that the antigens Spike and nucleocapsid have low toxicity in the body.

**Example 4. Immunogenicity evaluation through antibody immune responses**

Immunogenicity evaluation through antibody immune responses was performed by an Enzyme-Linked Immunosorbent Assay (ELISA) test method.

**Experiment method**

An ELISA plate was coated with 1 μg/ml of 1x phosphate buffered saline (PBS) containing a recombinant antigen and incubated overnight at 4°C. The coated plate was added with a blocking buffer (seracare, Cat.5140-0011) and then incubated at 37°C for 2 hours. After incubation, the plate was washed with 1x PBS containing 5% Tween 20, and the mouse serum was serially diluted and incubated at 37°C for 2 hours. The mouse serum was obtained from mice that were intramuscularly inoculated (twice at 2-week intervals) with the plasmid pGO-1002 prepared in Example 1 through electroporation inoculation and then delivered by electroporation. After incubation, the plate was washed with 1x PBS, incubated by adding a primary antibody binding to Spike or ORF3a, and then washed with 1x PBS. The plate was treated with a forse eadish peroxidase (HRP)-binding Goat anti-Mouse IgG (H+L) secondary antibody (invitrogen, Cat. 31430) (1 : 2,500), and then incubated at room temperature for 1 hour. Thereafter, the plate was washed with 1x PBS, and the plate was developed using an ABTS Peroxidase Substrate System (KPL, cat. 5120-0032) and treated with an ABTS Peroxidase Stop Solution (KPL, cat. 5150-0017), and then the reaction was stopped. The plate was read at 405 nm within 30 minutes. Synergy HTX (BioTek Instruments, Highland Park, VT) was used for reading the plate. Cutoff values were calculated as follows.

- cutoffs: Day 0 of each group; (each value - blank) Avg +(2.5 × SD)

Normalization was performed with the mouse serum inoculated with the plasmids pGLS-Spike and pGLS-ORF3a.

As a control, serum collected from mice inoculated with the plasmids pGLS-101 (negative control) and pGLS-Spike (positive control) was used.

**Measurement of Spike antigen-specific binding antibody titer**

In order to measure a Spike antigen-specific binding antibody titer, mouse serum was collected before plasmid inoculation (D0) and after 2 weeks (Wk2) and 4 weeks (Wk4). The Spike antigen-specific binding antibody titer was measured through ELISA using the collected serum and a primary antibody binding to Spike of the mice. The results of measuring the Spike antigen-specific binding antibody titer were illustrated in FIG. 4.

As illustrated in FIG. 4, it was confirmed that the experimental group (pGLS-Spike-ORF3a) inoculated with the plasmid pGO-1002 had a Spike antigen-specific binding antibody titer similar to that of a positive control (pGLS-Spike).

**Measurement of ORF3a antigen-specific binding antibody titer**

In order to measure a ORF3a antigen-specific binding antibody titer, mouse serum was collected before plasmid inoculation (D0) and after 2 weeks (Wk2) and 4 weeks (Wk4). The ORF3a antigen-specific binding antibody titer was measured through ELISA using the collected serum and a primary antibody binding to ORF3a of the mice. The results of measuring the ORF3a antigen-specific binding antibody titer were illustrated in FIG. 5.

As illustrated in FIG. 5, it was confirmed that the experimental group (pGLS-Spike-ORF3a) inoculated with the plasmid pGO-1002 had a significantly higher ORF3a antigen-specific binding antibody titer than that of the negative control (pGLS-101).

These results indicate that the Spike and ORF3a proteins expressed by the plasmid pGO-1002 induce sufficient immune responses, respectively.

In addition, the experiment was performed in the same manner as above using the bicistronic vector including the genes encoding Spike and nucleocapsid prepared in Example 1.

As a result, it was confirmed that the proteins (that is, Spike and nucleocapsid) expressed by the bicistronic vector including the genes encoding Spike and nucleocapsid prepared in Example 1 induced sufficient immune responses, respectively.

**Example 5. Immunogenicity evaluation through T-cell immune responses**

In order to remove a virus from the body, strong antibody responses and T-cell immune responses are simultaneously required. An experiment was performed to confirm whether Spike and ORF3a of the present invention induce T-cell immune responses as well as immunity by antibody formation. Immunogenicity evaluation through T-cell immune responses was performed by enzymelinked immuno-spot (ELISpot) assay.

**Experiment method**

The plasmid pGO-1002 was intramuscularly inoculated into mice through electroporation inoculation, and the inoculation was performed twice at 2-week intervals. The mice were sacrificed to obtain spleens. Single splenocytes were individually collected from mouse-derived spleens in an R10 medium to prepare a single cell suspension. The R10 medium refers to an RPMI1640 medium supplemented with 10% FBS and penicillin/streptomycin. The cell suspension was centrifuged to obtain a cell pellet, and the cell pellet was resuspended in 5 ml of an ACK lysis buffer (Life Technologies, cat. A1049201) and incubated for 8 minutes. To stop the reaction after incubation, an R10 medium was added to a tube containing the cells, and then centrifuged at 1400 rpm for 5 minutes to obtain a cell pellet. The cell pellet was resuspended in the R10 medium to prepare a cell suspension.

The ELISpot assay was performed using a mouse IFN-$\gamma$ ELISpotPLUS plate (MABTECH, cat. 33212H). For blocking, the R10 medium was added to a 96-well ELISpot plate coated with a capture antibody, and then incubated at room temperature for 1 hour. 200,000 mouse splenocytes were plated in each well of the blocked ELISpot plate. The plated splenocytes were stimulated with an overlapping long peptide (OLP) pool for 14 hours. The cells were stimulated with 1 $\mu$g/ml of each peptide per well in the R10 medium. Spots expressed after cell stimulation were developed according to a manufacturer's manual. A mixture of R10 medium and 1% DMSO and concanavaline A (ConA) were used as negative and positive controls, respectively. The spots were scanned and quantified with an ImmunoSpot CTL reader. A spot-forming unit (SFU) per 1,000,000 cells was calculated by subtracting the values of wells treated with the mixture of R10 medium and 1% DMSO. The cutoff values were calculated as follows: CUTOFF = MEAN of Spot numbers in OLPs + (Standard deviation of spot numbers in OLPs * 3). Non-vaccinated splenocytes were used as a sample for cutoff values.

The OLP pool was an OLP pool derived from a Spike or ORF3a protein of SARS CoV-2; or a combination thereof; Splenocyte collected from mice inoculated with the plasmids pGLS-101 (negative control) and pGLS-Spike (positive control) was used as a control.

**Evaluation of T-cell immune responses to Spike OLP**

T-cell immune responses of splenocytes stimulated with Spike OLP were evaluated by ELISpot analysis. In the evaluation of the T-cell immune responses, S1_OLP1 as 65 OLP pools corresponding to Spike V16-V350; S1_OLP2 as 65 OLP pools corresponding to Spike Y351-Q675; S2_OLP1 as 60 OLP pools corresponding to Spike I666-S975; S2_OLP2 as 60 OLP pools corresponding to Spike L966-T1273; and ORF3_OLP1 as 53 OLP pools corresponding to ORF3a M1-L275 were used. The OLP pool consists of a 15 mer-length peptide in which 10 amino acids are duplicated. The results of evaluating T-cell immune responses to Spike OLP were illustrated in FIG. 6.

As illustrated in FIG. 6, it was confirmed that the experimental group (pGLS-Spike-ORF3a) inoculated with the plasmid pGO-1002 effectively induced T-cell immune responses to Spike OLP compared to the positive control (pGLS-Spike).

**Evaluation of T-cell immune responses to ORF3a OLP**

T-cell immune responses of splenocytes stimulated with ORF3a OLP were evaluated through ELISpot analysis, and the results were illustrated in FIG. 7.

As illustrated in FIG. 7, the experimental group (pGLS-Spike-ORF3a) inoculated with the plasmid pGO-1002 effectively induced T-cell immune responses to ORF3a OLP.

**Evaluation of T-cell immune responses to Spike OLP and ORF3a OLP**

T-cell immune responses of splenocytes stimulated with Spike OLP and ORF3a OLP were evaluated by ELISpot analysis. In the experimental group, the plasmid pGO-1002 was administered to mice through intramuscular injection and electroporation. A control was a group administered with the plasmid pGLS101 by intramuscular injection and electroporation. The results of evaluating T-cell immune responses to Spike OLP and ORF3a OLP were illustrated in FIG. 8.

As illustrated in FIG. 8, it was confirmed that the experimental group (pGLS-Spike-ORF3a) inoculated with the plasmid pGO-1002 effectively induced T-cell immune responses by the expressed proteins Spike and ORF3a, respectively.

In addition, the experiment was performed in the same manner as above using the bicistronic vector including the genes encoding Spike and nucleocapsid prepared in Example 1.

As a result, it was confirmed that the experimental group inoculated with the bicistronic vector including the genes encoding Spike and nucleocapsid prepared in Example 1 showed a significant effect on T-cell immune responses compared to the positive control.

**Example 6. Evaluation of neutralizing antibody titer through plaque reduction neutralization assay**

A neutralizing antibody titer was evaluated through a $PRNT_{50}$ test using a neutralizing antibody of inhibiting Live SARS-CoV-2. Specifically, SARS-CoV-2/South Korea/2020 isolation and neutralization analysis was performed by the Korea Centers for Disease Control and Prevention. A neutralizing virus titer was measured in heat-inactivated serum samples for 30 minutes at 56°C. An experimental serum was obtained from mice inoculated (twice at 2-week intervals) with the plasmid pGO-1002 prepared in Example 1 through electroporation after intramuscular inoculation and a control serum was obtained from mice inoculated with the plasmid pGLS-101 or pGLS-Spike. SARS-CoV-2 (BetaCoV/Korea/KCDC03/2020) (60 pfu $ml^{-1}$) was mixed with a DMEM medium (Invitrogen, Carlsbad, USA) containing 10% FBS, 100 IU $ml^{-1}$ penicillin and 100 $\mu g\ ml^{-1}$ streptomycin at 50 : 50 and serially serum-diluted from 1 : 4 to 1 : 2040 in a 96-well U bottomed plate. The plate was incubated for 1 hour in a humidified box at 37°C and then a virus was inoculated in a 12-well plate (seeded with $2.5 \times 10^5$ Vero cells (ATCC, CCL-81) per well) in a DMEM (Invitrogen, Carlsbad, USA) (containing 10% FBS, 100 IU $ml^{-1}$ penicillin, 100 $\mu g\ ml^{-1}$ streptomycin). The virus was adsorbed for 1 hour at 37°C and overlaid with a plaque assay overlay medium (final 2x MEM/1.5% Agar/4% FBS). Thereafter, the virus was incubated for 3 days at 37°C in a humidified box. The cultured plate was fixed and stained to count plaques. A cytopathic effect (CPE) of each well was recorded under a microscope, and the neutralization titer was calculated using dilution water in a 50% protection condition. Experimental results were illustrated in FIGS. 9 and 10.

As illustrated in FIGS. 9 and 10, it was confirmed that the experimental group (pGLS-Spike-ORF3a) inoculated with the plasmid pGO-1002 prepared in Example 1 showed excellent neutralizing antibody immune responses similar to that of a pGLS-Spike inoculated group.

In addition, the experiment was performed in the same manner as above using the bicistronic vector including the genes encoding Spike and nucleocapsid prepared in Example 1.

As a result, it was confirmed that the experimental group inoculated with the bicistronic vector including the genes encoding Spike and nucleocapsid prepared in Example 1 had a high neutralizing antibody titer.

**Example 7. Evaluation of ACE receptor competition**

Circulating neutralizing antibodies against SARS-CoV-2 that blocked the interaction between a receptor-binding domain of a virus spike glycoprotein (RBD) and an ACE2 cell surface receptor (GenScript cat# L00847) were detected.

Control and experimental serums diluted in separate tubes were mixed with a diluted HRP-RBD solution in a 1:1 volume ratio. The experimental serum was obtained from mice intramuscularly inoculated (twice at 2-week intervals) with the plasmid pGO-1002 prepared in Example 1 through electroporation inoculation and the control serum was obtained from mice inoculated with the plasmid pGLS-101 or pGLS-Spike. The mixture was incubated at 37°C for 30 minutes. 100 $\mu$l each of the control was added into each well of the plate, and incubated at 37°C for 30 minutes by covering the plate with a plate sealer. The plate sealer was removed, and then the plate was washed 4 times with 260 $\mu$l of a 1X washing solution. To remove the liquid remaining in the wells after washing, the plate was tapped on a paper towel. Each well was added with 100 $\mu$l of a TMB solution, incubated in a dark room under a condition of 20 to 25°C for 15 minutes, and added with 50 $\mu$l of a stop solution and then the reaction was terminated. Absorbance was measured at 450 nm using a microtiter plate reader. The cutoff values were based on validation of a GenScript panel against confirmed COVID-19 patient serum and healthy control serum, and calculated using the following Equation.

$$\text{Inhibition} = (1 - \text{OD Value of sample/OD value of Negative Control}) * 100\%$$

Interpretation of the cutoff values was illustrated in Table 1.

[Table 1]

| Items | Cutoff | Result | Interpretation |
|---|---|---|---|
| SARS CoV-2 neutralizing antibody test | > 20% | Positive | SARS-CoV2 neutralizing antibody detected |
| | < 20% | Negative | No detectable SARS CoV-2 neutralizing antibody |

Results of evaluating ACE receptor competition were illustrated in FIG. 11. As illustrated in FIG. 11, it was confirmed that the experimental group (pGLS-Spike-ORF3a) inoculated with the plasmid pGO-1002 prepared in Example 1 had a significantly high competitive binding ability with a hACE2 protein.

In addition, the experiment was performed in the same manner as above using the bicistronic vector including the genes encoding Spike and nucleocapsid prepared in Example 1.

As a result, it was confirmed that the experimental group inoculated with the bicistronic vector including the genes encoding Spike and nucleocapsid prepared in Example 1 had a high competitive binding ability with the hACE2 protein.

Overall, the present inventors prepared a bicistronic vector including Spike and ORF3a genes; and a bicistronic vector including Spike and nucleocapsid genes, which were genes encoding a DNA vaccine, that is, antigen protein against SARS CoV-2. The bicistronic vector produced two antigen proteins through expression and post-translational modification in vivo, and the produced antigenic proteins exhibited significant immunogenicity, and more specifically, it was confirmed that the bicistronic vector may not only induce distinct antibody immune responses, but also induce strong T-cell immune responses to two types of antigens, respectively. Therefore, the bicistronic vector according to the present invention may be variously used in the field of prevention of SARS-CoV-2 virus infection.

Hereinafter, the present invention will be described in more detail with reference to Preparation Example. Preparation Example is only illustrative of the present invention, and the scope of the present invention is not construed to be limited to Preparation Example.

**Preparation Example 1. Vaccine composition**

Plasmid pGO-1002
Sodium Citrate hydrate
Sodium Chloride
Sterile water for injection

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. A vaccine composition for preventing severe acute respiratory syndrome coronavirus 2 infection comprising:

   (a) a SARS-CoV-2 Spike protein or a gene encoding the SARS-CoV-2 Spike protein; and
   (b) a SARS-CoV-2 ORF3a protein or a gene encoding the SARS-CoV-2 ORF3a; or a SARS-CoV-2 nucleocapsid or a gene encoding the SARS-CoV-2 nucleocapsid.

2. An immunogenic composition for severe acute respiratory syndrome coronavirus 2 comprising:

   (a) a SARS-CoV-2 Spike protein or a gene encoding the SARS-CoV-2 Spike protein; and
   (b) a SARS-CoV-2 ORF3a protein or a gene encoding the SARS-CoV-2 ORF3a; or a SARS-CoV-2 nucleocapsid or a gene encoding the SARS-CoV-2 nucleocapsid.

3. The composition of claim 1 or 2, wherein the gene encoding the Spike, ORF3a or nucleocapsid protein is optimized with human codons.

4. The composition of claim 3, wherein the gene encoding the Spike protein is represented by a nucleotide sequence of SEQ ID NO: 1 or 3.

5. The composition of claim 3, wherein the gene encoding the ORF3a protein is represented by a nucleotide sequence of SEQ ID NO: 2.

6. The composition of claim 3, wherein the gene encoding the nucleocapsid protein is represented by a nucleotide sequence of SEQ ID NO: 4.

7. The composition of claim 1 or 2, wherein (a) the gene encoding the Spike protein; and (b) the gene encoding ORF3a or nucleocapsid protein are included in a bicistronic expression vector.

8. The composition of claim 1 or 2, wherein the composition is for two doses.

9. The composition of claim 1 or 2, wherein the composition simultaneously induces the immunogenicity against (a) the Spike protein; and (b) the ORF3a or nucleocapsid protein.

10. The composition of claim 1 or 2, wherein the composition simultaneously induces antibody responses and T-cell immune responses.

11. A bicistronic expression cassette comprising:

    (a) a gene encoding a SARS-CoV-2 Spike protein; and
    (b) a gene encoding a SARS-CoV-2 ORF3a protein; or a gene encoding a SARS-CoV-2 nucleocapsid protein.

12. The bicistronic expression cassette of claim 11, wherein the gene encoding the Spike, ORF3a or nucleocapsid protein is optimized with human codons.

13. The bicistronic expression cassette of claim 12, wherein the gene encoding the Spike protein is represented by a nucleotide sequence of SEQ ID NO: 1 or 3.

14. The bicistronic expression cassette of claim 12, wherein the gene encoding the ORF3a protein is represented by a nucleotide sequence of SEQ ID NO: 2.

15. The bicistronic expression cassette of claim 12, wherein the gene encoding the nucleocapsid protein is represented by a nucleotide sequence of SEQ ID NO: 4.

16. The bicistronic expression cassette of claim 11, further comprising:
    one or more components selected from the group consisting of a CMV promoter, a Kozak sequence, an IgE leader sequence, a furin recognition site, a GSG-T2A cleavage site, a BGH polyadenylation signal, a kanamycin resistance gene and a pUC origin.

17. The bicistronic expression cassette of claim 16, wherein the bicistronic expression cassette is represented by the following cleavage map.

[Cleavage map]

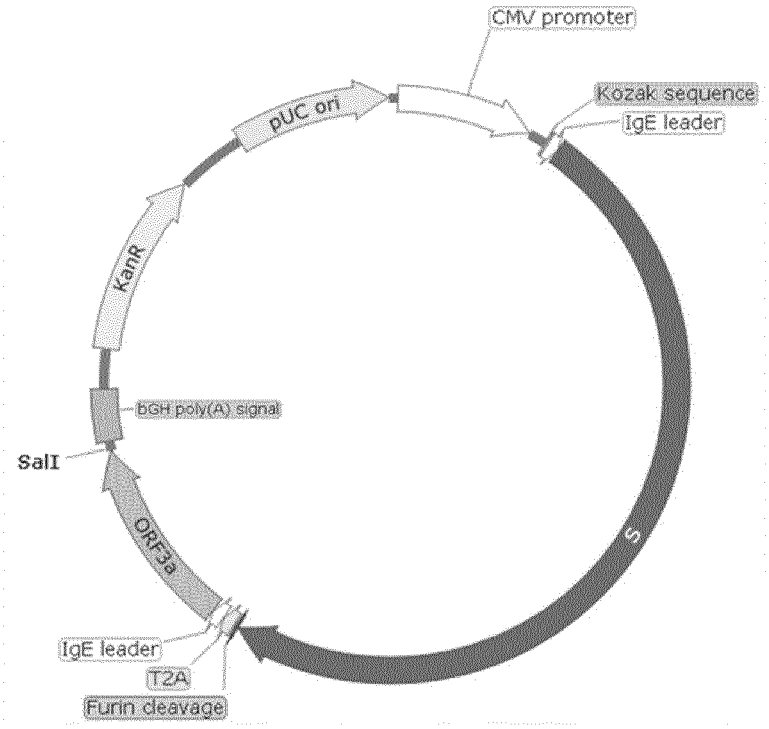


18. A vaccine composition for preventing severe acute respiratory syndrome coronavirus 2 infection comprising the bicistronic expression cassette according to claim 11.

19. An immunogenic composition for severe acute respiratory syndrome coronavirus 2 comprising the bicistronic expression cassette according to claim 11.

20. The composition of claim 18 or 19, wherein the composition is administered by at least one selected from the group consisting of intramuscular, intravenous, subcutaneous, intradermal and intranasal routes.

21. The composition of claim 18 or 19, wherein the composition is administered by at least one method selected from the group consisting of electroporation, gene gun, and jet injection.

22. The composition of claim 18 or 19, wherein the composition is administered intramuscularly through electroporation.

23. The composition of claim 18 or 19, wherein the composition is administered through a liposome-mediated delivery method.

24. A method for preventing severe acute respiratory syndrome coronavirus 2 infection comprising treating the bicistronic expression cassette according to claim 11 to a subject.

【Figure 1a】

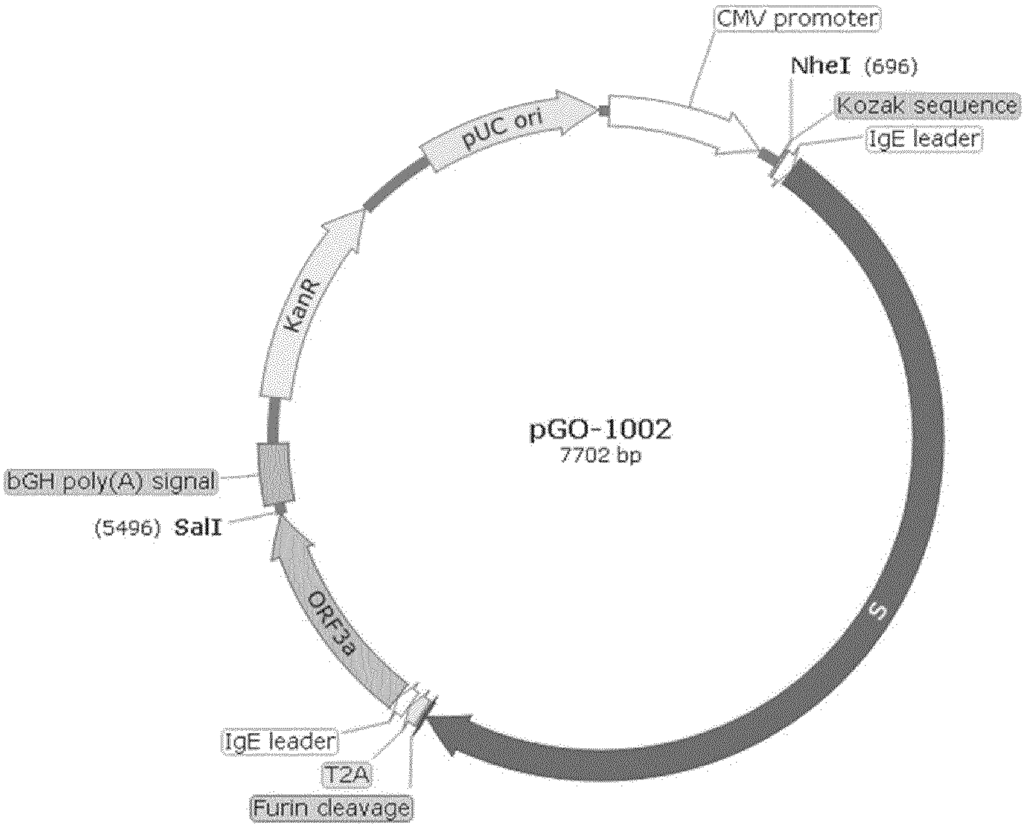
CMV promoter
NheI (696)
Kozak sequence
IgE leader
pUC ori
KanR
pGO-1002
7702 bp
bGH poly(A) signal
(5496) SalI
ORF3a
S
IgE leader
T2A
Furin cleavage

【Figure 1b】

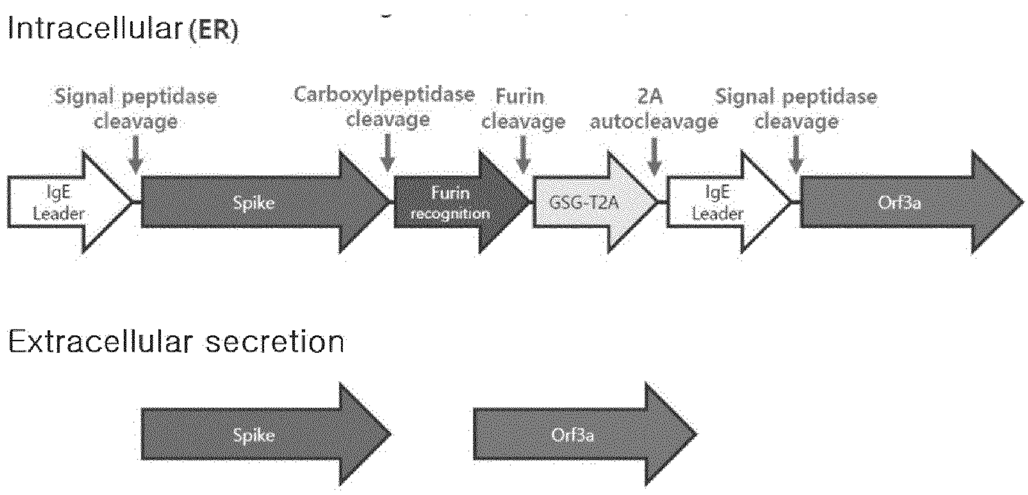
Intracellular (ER)
Signal peptidase cleavage
Carboxylpeptidase cleavage
Furin cleavage
2A autocleavage
Signal peptidase cleavage
IgE Leader
Spike
Furin recognition
GSG-T2A
IgE Leader
Orf3a
Extracellular secretion
Spike
Orf3a

【Figure 2a】

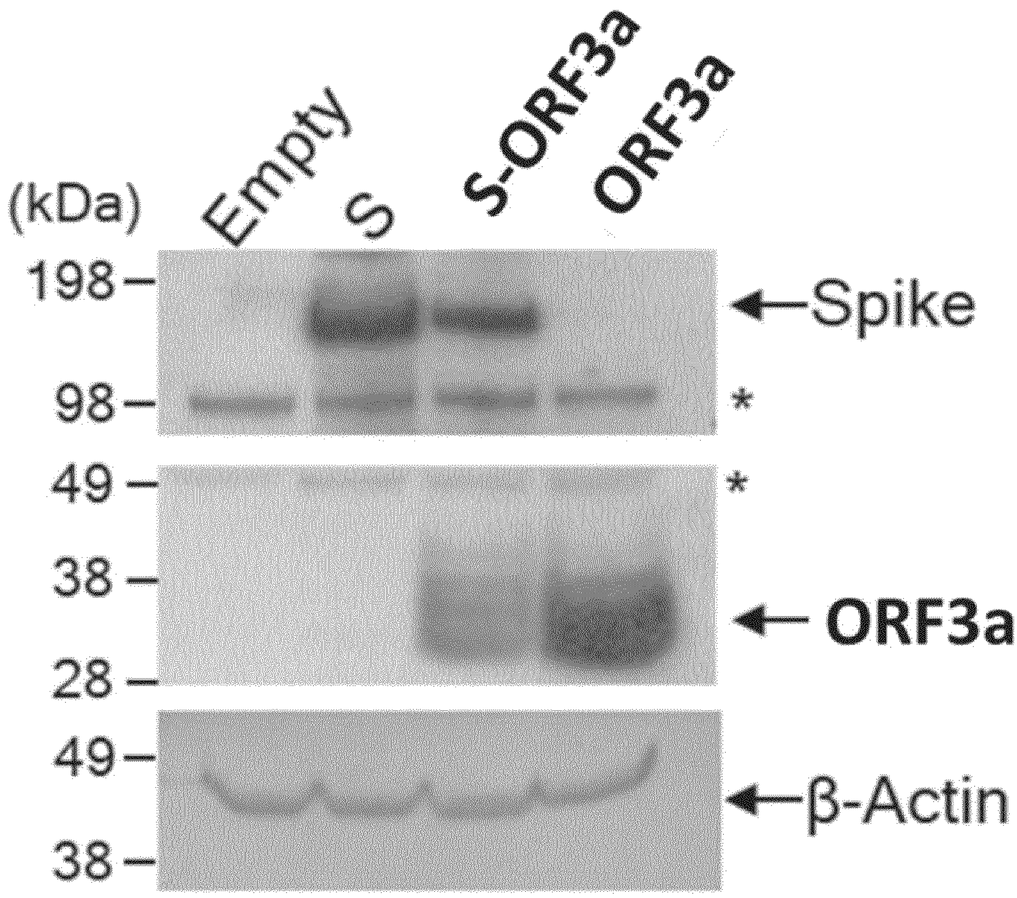


- Empty : pGLS101
- S : pGLS-Spike
- S-Orf3a : pGLS-Spike-ORF3a
- Orf3a : pGLS-ORF3a

【Figure 2b】

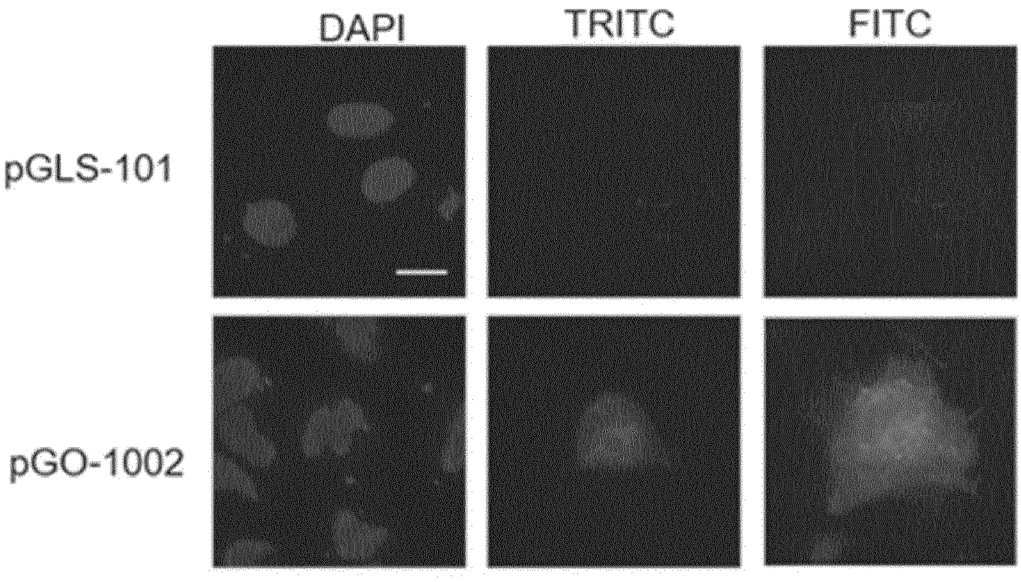

【Figure 3】

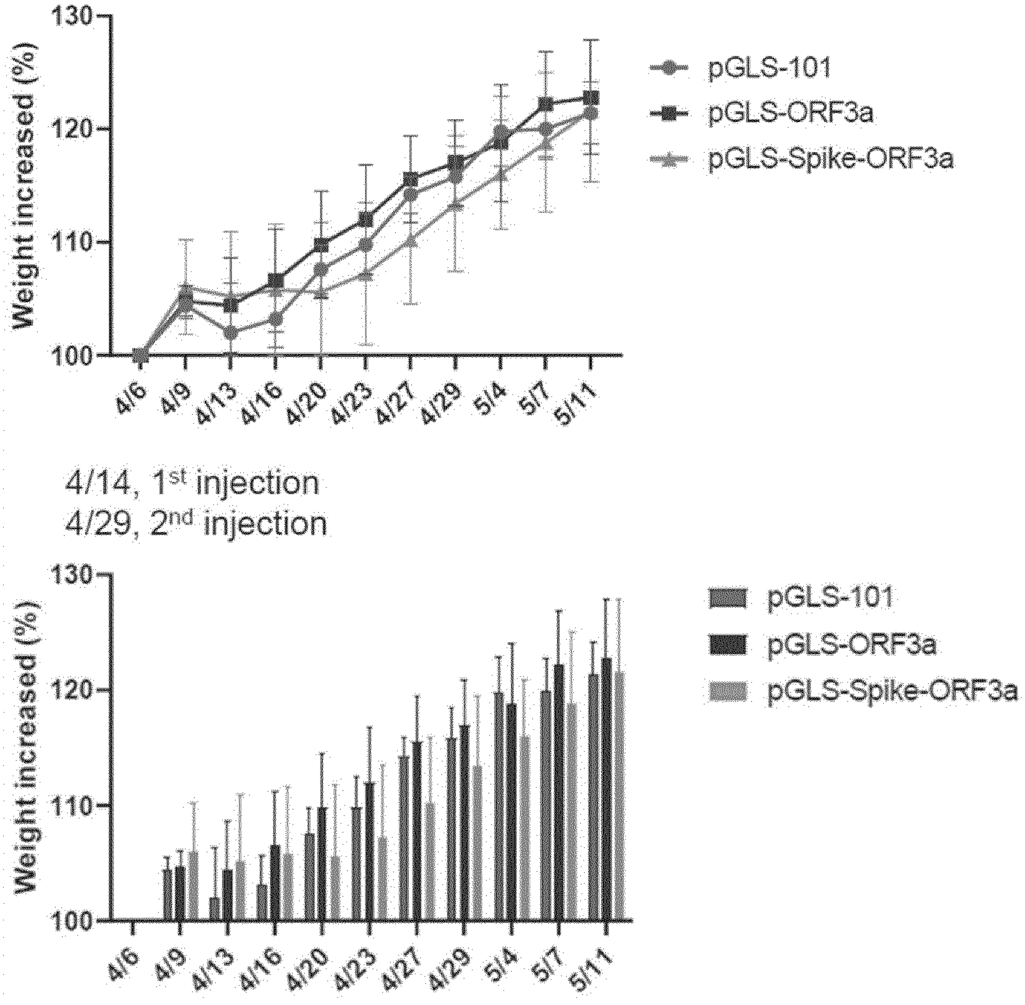
130
120
110
100
Weight increased (%)
pGLS-101
pGLS-ORF3a
pGLS-Spike-ORF3a
4/6
4/9
4/13
4/16
4/20
4/23
4/27
4/29
5/4
5/7
5/11
4/14, 1st injection
4/29, 2nd injection
130
120
110
100
Weight increased (%)
pGLS-101
pGLS-ORF3a
pGLS-Spike-ORF3a
4/6
4/9
4/13
4/16
4/20
4/23
4/27
4/29
5/4
5/7
5/11

【Figure 4】

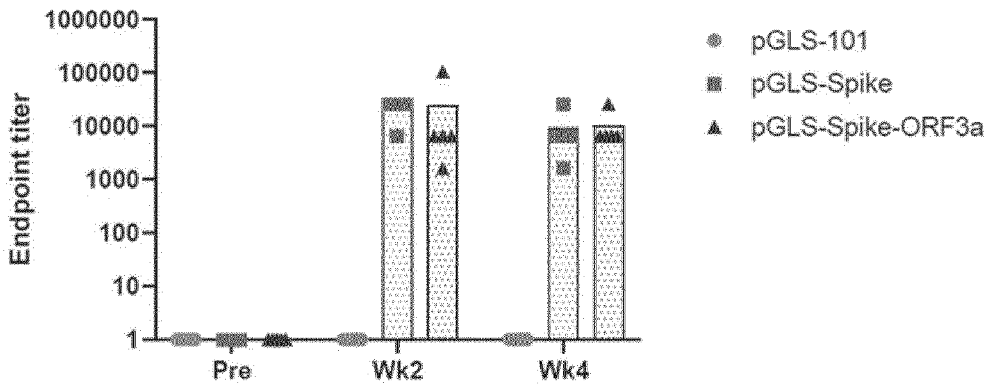
Endpoint titer
1000000
100000
10000
1000
100
10
1
Pre
Wk2
Wk4
pGLS-101
pGLS-Spike
pGLS-Spike-ORF3a

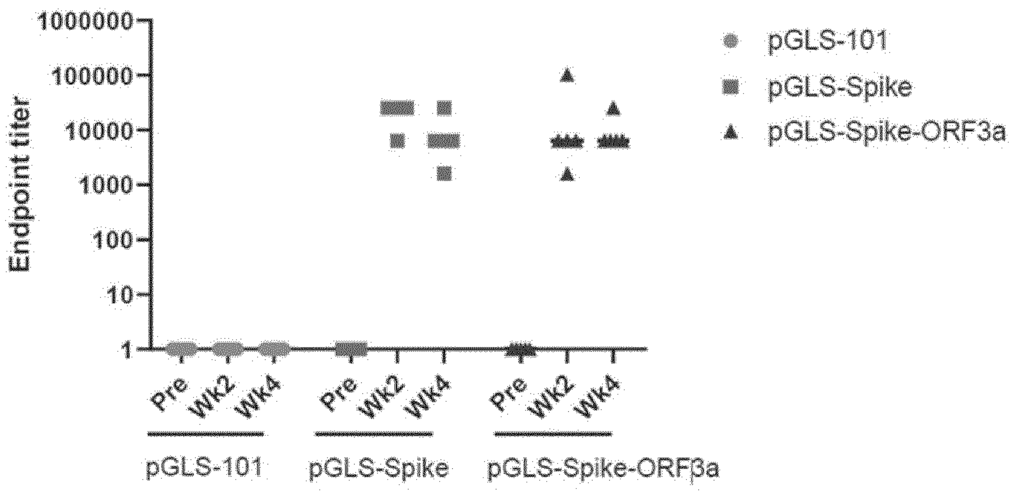
Endpoint titer
1000000
100000
10000
1000
100
10
1
Pre
Wk2
Wk4
pGLS-101
pGLS-Spike
pGLS-Spike-ORFβa
pGLS-Spike-ORF3a

【Figure 5】

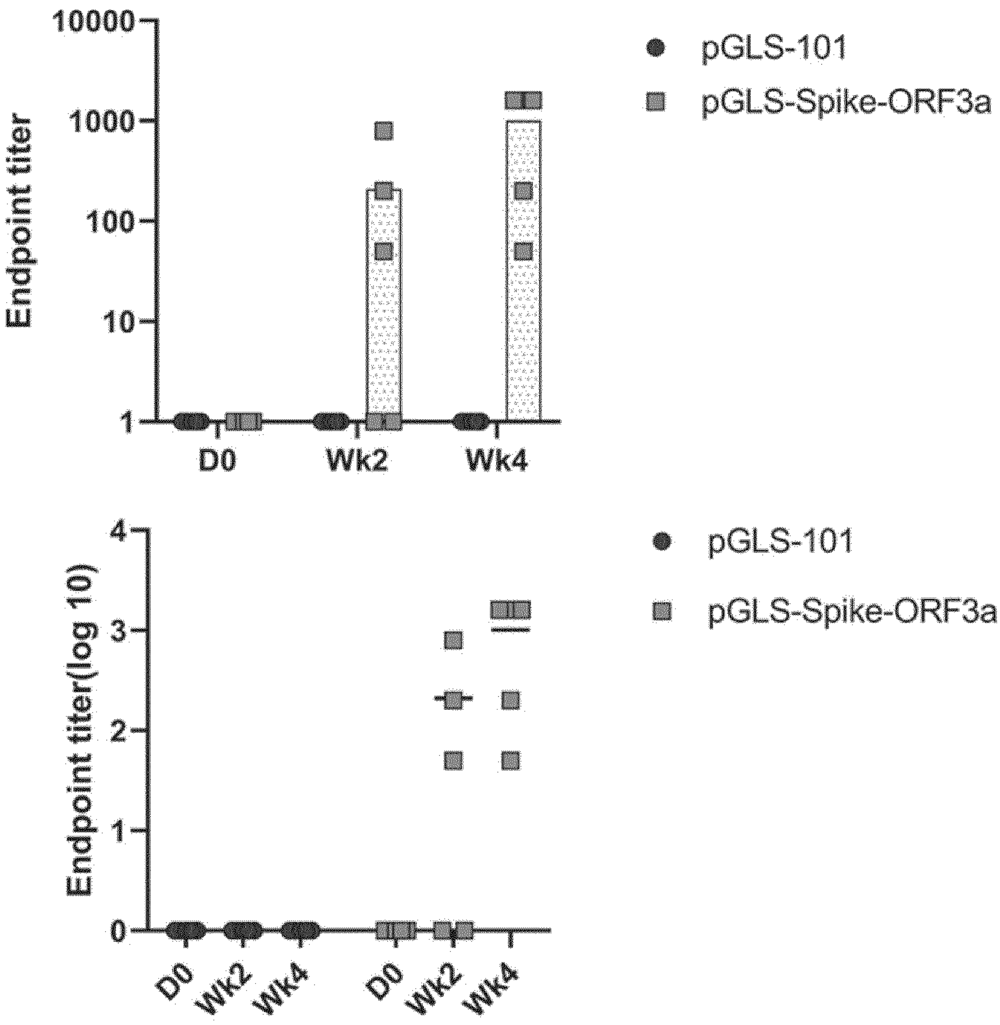
10000
1000
100
10
1
Endpoint titer
D0
Wk2
Wk4
pGLS-101
pGLS-Spike-ORF3a
4
3
2
1
0
Endpoint titer(log 10)
D0
Wk2
Wk4
D0
Wk2
Wk4
pGLS-101
pGLS-Spike-ORF3a

【Figure 6】

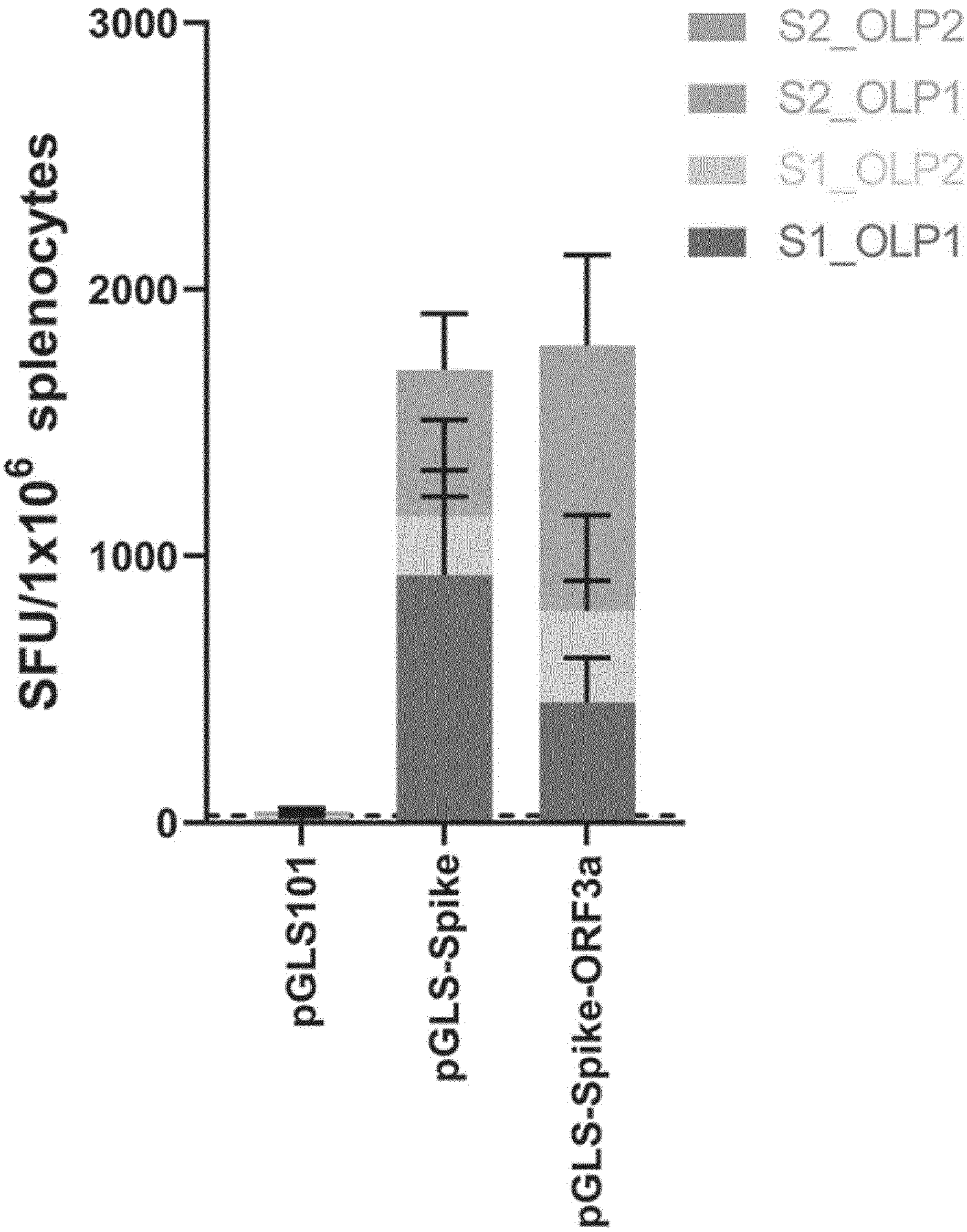
3000
2000
1000
0
SFU/1x10^6 splenocytes
S2_OLP2
S2_OLP1
S1_OLP2
S1_OLP1
pGLS101
pGLS-Spike
pGLS-Spike-ORF3a

【Figure 7】

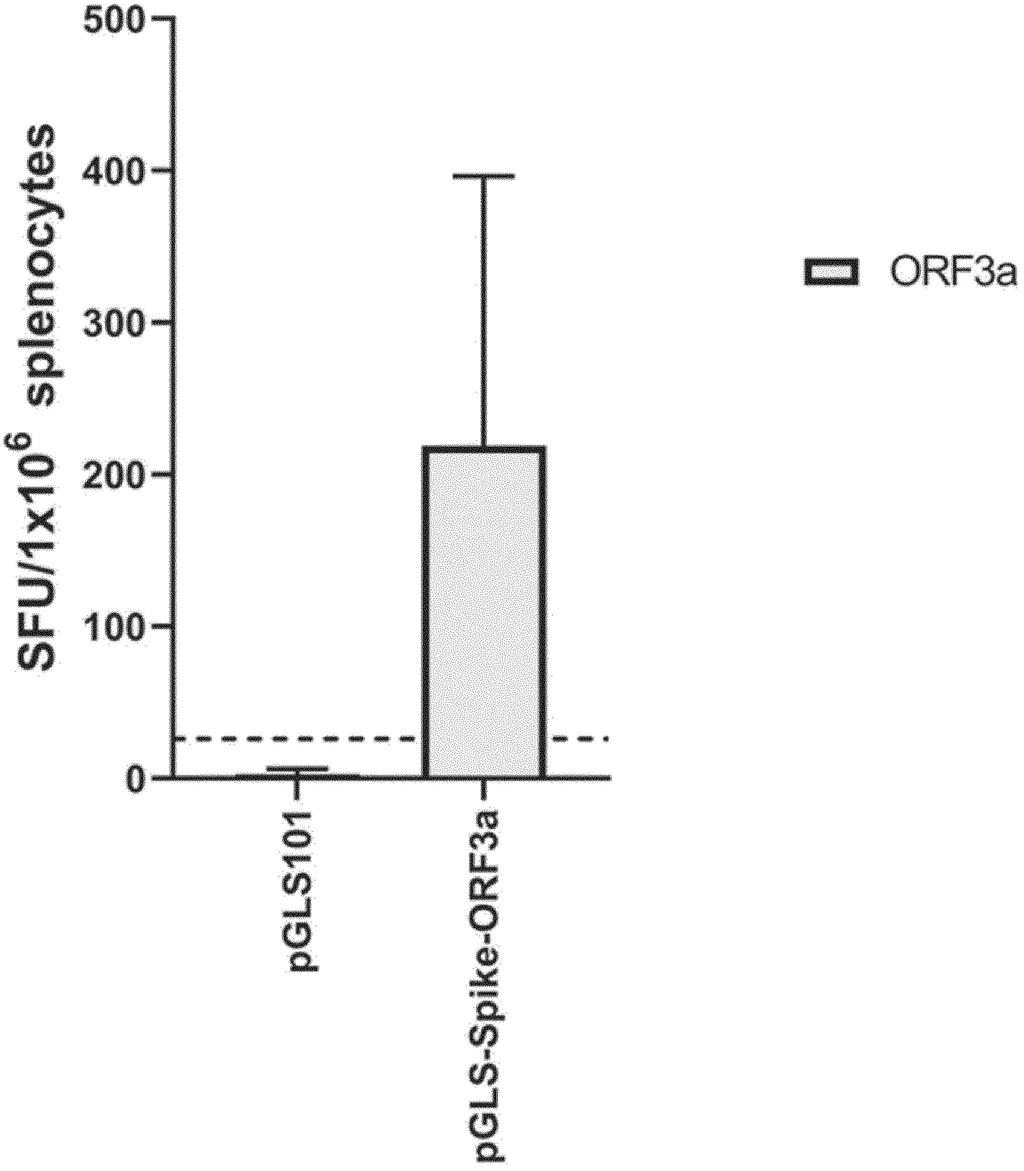
SFU/1x10^6 splenocytes
500
400
300
200
100
0
pGLS101
pGLS-Spike-ORF3a
ORF3a

【Figure 8】

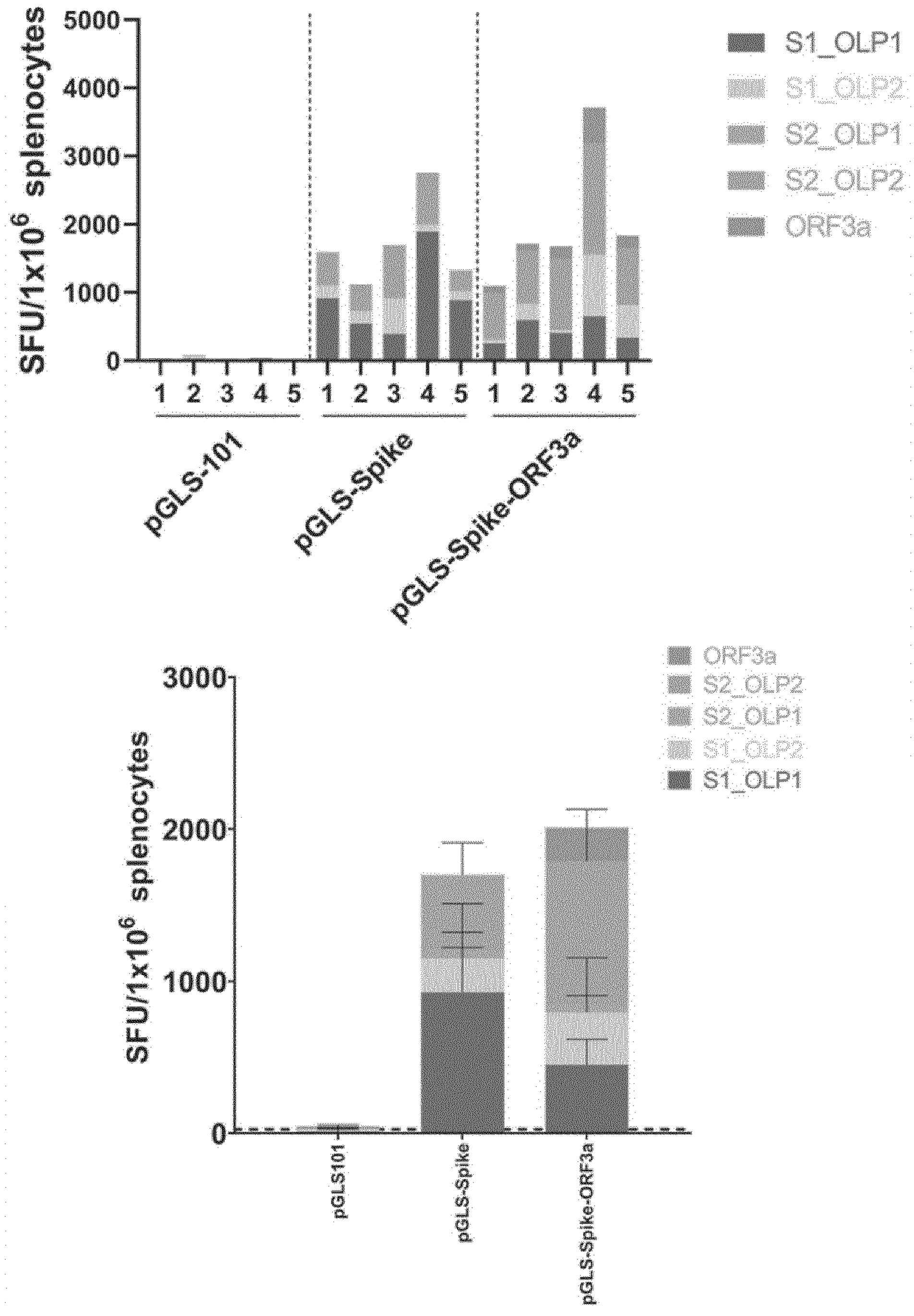
SFU/1x10^6 splenocytes
5000
4000
3000
2000
1000
0
1 2 3 4 5 1 2 3 4 5 1 2 3 4 5
pGLS-101
pGLS-Spike
pGLS-Spike-ORF3a
S1_OLP1
S1_OLP2
S2_OLP1
S2_OLP2
ORF3a
SFU/1x10^6 splenocytes
3000
2000
1000
0
pGLS101
pGLS-Spike
pGLS-Spike-ORF3a
ORF3a
S2_OLP2
S2_OLP1
S1_OLP2
S1_OLP1

【Figure 9】

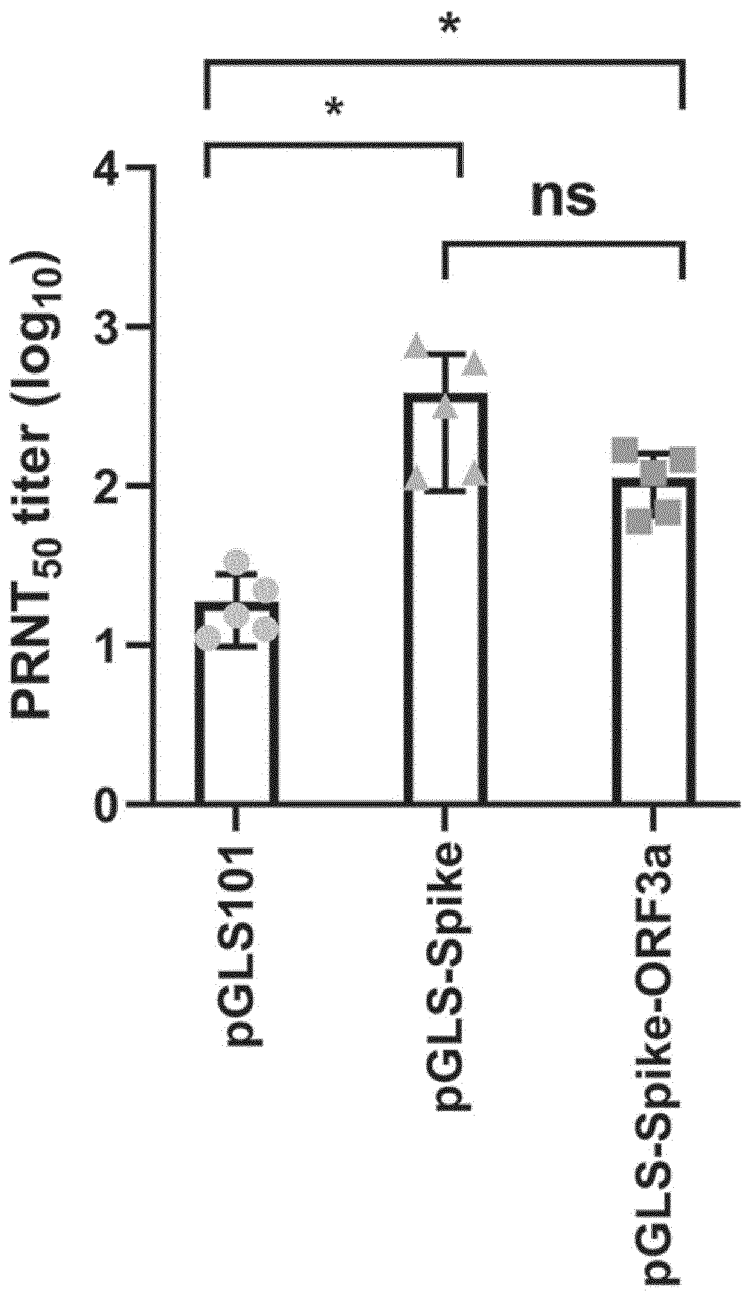
*
*
ns
4
3
2
1
0
PRNT50 titer (log10)
pGLS101
pGLS-Spike
pGLS-Spike-ORF3a

【Figure 10】

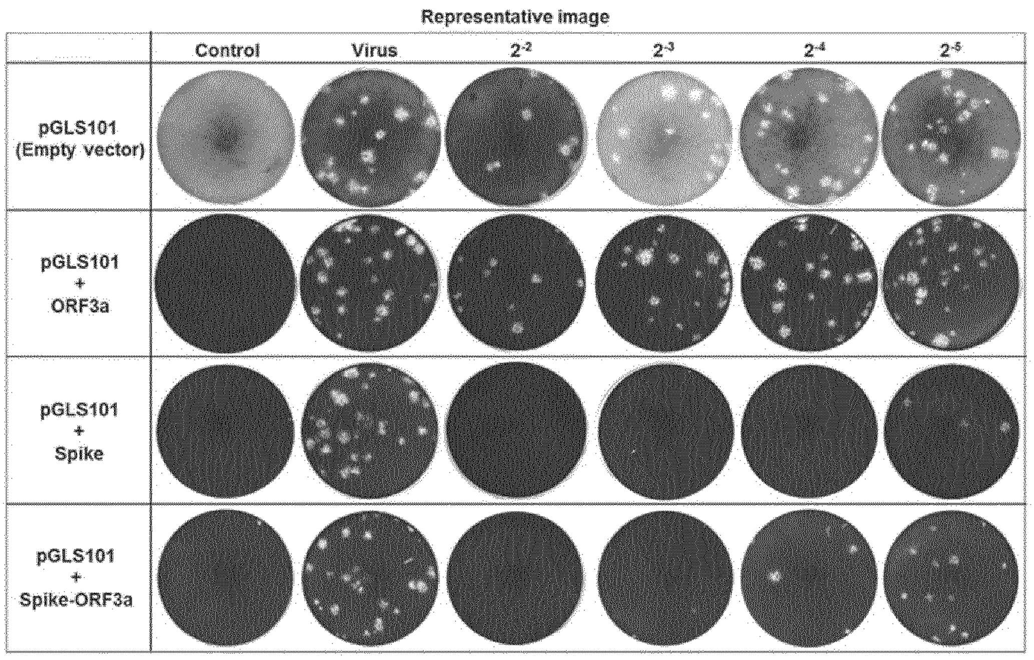


| Neutralizing Ab titer | | | | | | | |
|---|---|---|---|---|---|---|---|
| | 1 | 2 | 3 | 4 | 5 | Mean | SD |
| pGLS101 | 16.57 | 7.71 | 11.04 | 5.53 | 6.31 | 9.43 | 4.51 |
| pGLS-ORF3a | 2.17 | 0.00 | 0.00 | 2.80 | 0.00 | 0.99 | 1.38 |
| pGLS-Spike | 149.33 | 28.44 | 30.40 | 80.00 | 192.00 | 96.04 | 72.77 |
| pGLS-Spike+ORF3a | 30.00 | 83.69 | 60.44 | 73.14 | 34.29 | 56.31 | 23.60 |

【Figure 11】

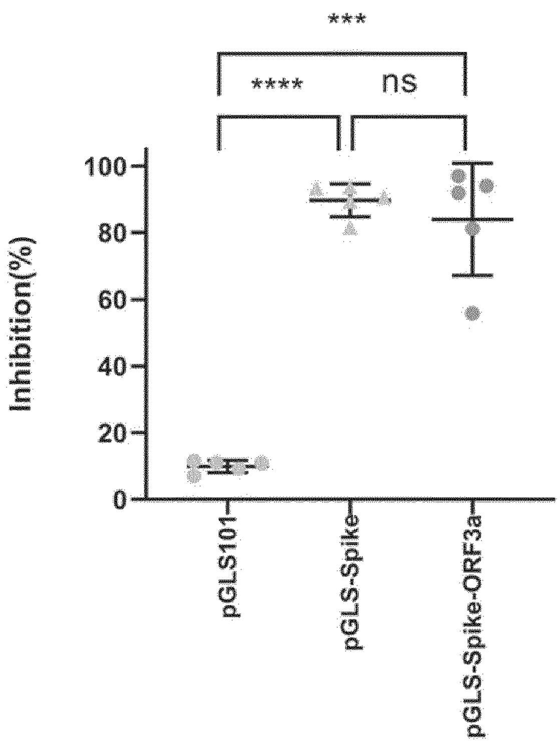


| summary | P Value |
|---|---|
| ns | No significantly different |
| *** | <0.001 |
| **** | <0.0001 |

**INTERNATIONAL SEARCH REPORT**

International application No.
**PCT/KR2021/009290**

**A. CLASSIFICATION OF SUBJECT MATTER**

**A61K 39/215**(2006.01)i; **A61P 31/14**(2006.01)i; **C07K 14/005**(2006.01)i

According to International Patent Classification (IPC) or to both national classification and IPC

**B. FIELDS SEARCHED**

Minimum documentation searched (classification system followed by classification symbols)

A61K 39/215(2006.01); C07K 19/00(2006.01); C12N 15/50(2006.01); C12N 15/62(2006.01); C12N 15/85(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 중증급성호흡기증후군 코로나바이러스 2 (SARS-CoV-2), 항원 (antigen), DNA 백신 (DNA vaccine), T세포 면역반응 (T cell immune response), 바이시스트로닉 발현 벡터 (bicistronic expression vector)

**C. DOCUMENTS CONSIDERED TO BE RELEVANT**

| Category* | Citation of document, with indication, where appropriate, of the relevant passages | Relevant to claim No. |
|---|---|---|
| X<br>A | CHIUPPESI, F. et al. Development of a Synthetic Poxvirus-Based SARS-CoV-2 Vaccine. bioRxiv. 2020, inner pp. 1-40 (publication date: 02 July 2020).<br>See abstract, inner pages 3, 7-10, 15 and 21, and Figures 4-6. | 1,2,7-11,16,18-23<br>3-6,12-15,17 |
| A | CN 111217919 A (SUN YAT-SEN UNIVERSITY) 02 June 2020 (2020-06-02)<br>See entire document. | 1-23 |
| A | KR 10-2003-0062118 A (LG LIFE SCIENCES LTD.) 23 July 2003 (2003-07-23)<br>See entire document. | 1-23 |
| A | SMITH, T. R. F. et al. Immunogenicity of a DNA vaccine candidate for COVID-19. Nature Communications. 2020, vol. 11, no. 2601, inner pp. 1-13 (published online: 20 May 2020).<br>See entire document. | 1-23 |
| A | CN 110951756 A (GUANGZHOU NBIOMED MEDICINE TECHNOLOGY CO., LTD.) 03 April 2020 (2020-04-03)<br>See entire document. | 1-23 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 October 2021** | **25 October 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2021/009290**

**C. DOCUMENTS CONSIDERED TO BE RELEVANT**

| Category* | Citation of document, with indication, where appropriate, of the relevant passages | Relevant to claim No. |
|---|---|---|
| PX | MAMEDOV, T. et al. Engineering, production and characterization of Spike and Nucleocapsid structural proteins of SARS–CoV-2 in Nicotiana benthamiana as vaccine candidates against COVID-19. bioRxiv. 2020, inner pp. 1-27 (published online: 30 December 2020). See abstract, inner pages 7-11 and Figures 3, 6 and 8. | 1,2,7-9,11,18-20 |

Form PCT/ISA/210 (second sheet) (July 2019)

**INTERNATIONAL SEARCH REPORT**

International application No.
**PCT/KR2021/009290**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**

International application No.
**PCT/KR2021/009290**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **24**
because they relate to subject matter not required to be searched by this Authority, namely:

Claim 24 pertains to a method for treatment of the human body by surgery or therapy, as well as a diagnostic method (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2021/009290**

| Patent document cited in search report | Publication date (day/month/year) | Patent family member(s) | Publication date (day/month/year) |
|---|---|---|---|
| CN 111217919 A | 02 June 2020 | CN 111217919 B | 01 December 2020 |
| KR 10-2003-0062118 A | 23 July 2003 | KR 10-0450266 B1 | 30 September 2004 |
| CN 110951756 A | 03 April 2020 | CN 110951756 B | 04 August 2020 |
| | | CN 110974950 A | 10 April 2020 |
| | | CN 110974950 B | 07 August 2020 |
| | | EP 3804751 A2 | 14 April 2021 |
| | | EP 3805392 A2 | 14 April 2021 |
| | | WO 2021-000968 A2 | 07 January 2021 |
| | | WO 2021-000968 A3 | 18 February 2021 |
| | | WO 2021-000969 A2 | 07 January 2021 |
| | | WO 2021-000969 A3 | 18 February 2021 |

Form PCT/ISA/210 (patent family annex) (July 2019)